(19) Europäisches Patentamt
European Patent Office
Office européen des brevets

(11) Numéro de publication: **0 168 296**
**B1**

# (12) FASCICULE DE BREVET EUROPÉEN

(45) Date de publication du fascicule du brevet: **02.08.89**

(51) Int. Cl.⁴: **C 07 D 249/14** // C07C133/10

(21) Numéro de dépôt: **85401161.6**

(22) Date de dépôt: **12.06.85**

## (54) Procédé de préparation de l'amino-3 triazole-1,2,4.

(30) Priorité: **20.06.84 FR 8409663**

(43) Date de publication de la demande: **15.01.86 Bulletin 86/3**

(45) Mention de la délivrance du brevet: **02.08.89 Bulletin 89/31**

(84) Etats contractants désignés: **AT BE CH DE FR GB IT LI NL**

(56) Documents cité:
**FR-A-1 300 971**

**CHEMICAL ABSTRACTS, vol. 76, no. 24, 12 juin 1972, page 473, no. 153796h, Columbus, Ohio, US**

**Le dossier contient des informations techniques présentées postérieurement au dépôt de la demande et ne figurant pas dans le présent fascicule.**

(73) Titulaire: **ATOCHEM, 4 & 8, Cours Michelet La Défense 10, F-92800 Puteaux (FR)**

(72) Inventeur: **Alicot, Michel, Route d'Arreau, F-65250 La Barthe de Neste (FR)**

(74) Mandataire: **Leboulenger, Jean, ATOCHEM Département Propriété Industrielle, F-92091 Paris la Défense 10 Cédex 42 (FR)**

**EP 0 168 296 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne la synthèse de l'amino-3 triazo le-1,2,4. Ce composé de formule:

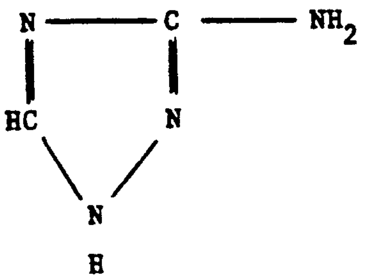

est utilisé principalement comme désherbant et, dans une moindre mesure, comme matière première de synthèse (plus particulièrement dans l'industrie des matières colorantes).

Industriellement, l'amino-3 triazole-1,2,4 est obtenu par réaction d'un sel d'aminoguanidine (le plus souvent, le bicarbonate) avec l'acide formique, puis cyclisation du formate d'aminoguanidine formé (brevet US-2 875 209). Dans un stade préliminaire, l'aminoguanidine est obtenue sous forme de sel soluble dans l'eau par condensation de l'hydrate d'hydrazine et de la cyanamide en présence d'un acide minéral (notamment $H_2SO_4$) et isolée sous forme de bicarbonate peu soluble par carbonatation en milieu alcalin (brevet FR-1 241 151).

Ce procédé industriel présente cependant de graves inconvénients. Son bilan économique est grevé par différents facteurs:

. Excès important de cyanamide, nécessaire pour effectuer la réaction complète de l'hydrate d'hydrazine;

. Utilisation d'un acide minéral et, par voie de conséquence, celle d'un agent alcalin au stade de la carbonatation;

. Consommation d'anhydride carbonique;

. Perte de rendement par solubilité partielle du bicarbonate d'aminoguanidine dans les eaux-mères et les eaux de lavage, dont le recyclage est impossible en raison en particulier de la presence d'ions minéraux;

. Effluents importants (tant en volume qu'en charge polluante) qu'il n'est pas possible d'épurer à un niveau acceptable.

En outre, l'amino-3 triazole-1,2,4 obtenu par ce procédé n'est pas d'une pureté suffisante: les solutions aqueuses ne présentent pas une bonne filtrabilité, ce qui est le signe de la présence de produits insolubles qui, bien qu'en faible quantité, sont d'autant plus gênants qu'ils se présentent le plus souvent sous forme de gel.

Dans la demande de brevet japonaise publiée sous le no. 7 208 820, on a proposé de préparer l'amino-3 triazole-1,2,4 par réaction de l'hydrate d'hydrazine et de la cyanamide en présence d'acide formique, cette opération étant effectuée en une seule étape (exemples 1 à 9) ou en deux étapes (exemple 10). Dans les deux cas, la pureté de l'amino-3 triazole-1,2,4 produit n'est pas satisfaisante, les solutions aqueuses développant des floculats plus ou moins gélatineux, difficiles à éliminer. D'autre part, le rendement obtenu en opérant comme à l'exemple 10 est trop faible pour rendre ce procédé économiquement viable.

Il a maintenant été trouvé un procédé permettant d'obtenir avec un rendement élevé un amino-3 triazole-1,2,4 d'excellente pureté (absence de floculat dans les solutions aqueuses) à partir d'hydrate d'hydrazine, de cyanamide et d'acide formique.

Le procédé selon l'invention qui consiste à faire réagir d'abord l'hydrate d'hydrazine, la cyanamide et l'acide formique, puis à cycliser séparément le formiate d'aminoguanidine est caractérisé en ce que:

a) on ajoute simultanément l'hydrate d'hydrazine et l'acide formique à la cyanamide de façon à maintenir la température entre 0°C et 10°C, de préference entre 0 et 5°C, tout en régulant le pH (mesuré à 4°C) entre 6 et 7, de préférence entre 6,3 et 6,5;

b) on porte ensuite le mélange à une température comprise entre 60 et 100°C, de préférence entre 75 et 85°C, et l'y maintient jusqu'à disparition de l'hydrate d'hydrazine, le pH (mesuré à 20°C) étant maintenu entre 7 et 8, de préférence entre 7,5 et 7,7, par addition d'acide formique;

c) on évapore ensuite partiellement la solution de formiate d'aminoguanidine ainsi obtenue à une température comprise entre 30 et 60°C, de préférence entre 35 et 45°C, jusqu'à ce que la teneur en solides soit comprise entre 100 et 700 g/l et de préférence voisine de 500 g/l;

d) on filtre la suspension et lave le formiate d'aminoguanidine cristallisé jusqu'à ce que sa teneur en dicyandiamide soit inférieure à 0,25 % par rapport au poids de formiate sec, les eaux-mères et une partie ou la totalité des eaux de lavage étant recyclées à l'évaporation; et

e) on chauffe le formiate d'aminoguanidine à une température comprise entre 110 et 200°C, de préférence entre 140 et 170°C.

Bien que les concentrations des trois matières premières ne soient pas critiques pour la réussite du procédé, on préfère employer l'hydrate d'hydrazine pur ou sous forme de solutions aqueuses ayant une concentration supérieure à 35 % en poids, ainsi que de l'acide formique pur ou sous forme de solutions aqueuses ayant une concentration supérieure à 70 % en poids. La cyanamide est de préférence engagée sous forme de solutions aqueuses dont la concentration est comprise entre 100 et 250 g/l; des concentrations différentes entrent dans le cadre de la présente invention: plus faibles, elles conduisent cependant à un milieu dilué en eau ce qui entraîne un bilan thermique défavorsble lors de l'évaporation; plus élevées, elles ont un effet défavorable sur la phase de lavage. On peut également engager la cyanamide sous forme de solutions dans un solvant organique, par exemple un alcool tel que le butanol.

Bien que le rapport molaire des trois réactifs puisse être celui correspondant à la stoechiométrie (une mole de cyanamide et une mole d'acide formique par mole d'hydrazine), il est avantageux, pour améliorer la vitesse de réaction, d'utiliser par rapport à l'hydrazine un excès molaire de cyanamide compris entre 0 et 5 %, preferentiellement compris entre 0 et 3 %, et un excès molaire d'acide formique compris entre 0 % et 8 %, de préference entre 2 et 6 %.

L'évaporation est de préférence effectuée sous vide entre 10 et 100 torr, en particulier entre 20 et 30 torr. Cette étape et celle subséquente de filtration et lavage sont de préférence réalisées en continu. Après une mise en régime durant laquelle, par suite du recyclage des eaux-mères et d'une partie ou de la totalité des eaux de lavage, la teneur en impuretés est laissée s'élever jusqu'à une valeur correspondant à une concentration en dicyandiamide comprise entre 5 et 15 %, de préférence entre 8 et 12 %, on réalise une purge en prélevant sur les eaux-mères une quantité de liquide telle qu'on élimine une quantité d'impuretés équivalente à celle de la solution de formiate arrivant à l'évaporateur. Le formiate d'aminoguanidine entraîné par cette purge peut éventuellement être récupéré et utilisé séparément pour une fabrication d'amino-3 triazole-1,2,4 de pureté moins élevée que celle visée par la présente invention.

L'exemple suivant illustre l'invention sans la limiter. Les pourcentages indiqués sont exprimés en poids.

**Exemple**

a) Dans un réacteur équipé d'une agitation, d'une mesure de pH asservie à l'introduction d'acide formique, d'une prise de température et d'un échangeur alimenté en liquide réfrigérant, on introduit sous agitation et sous couverture d'azote 31330 litres d'une solution aqueuse de cyanamide à 160 g/l et 3 kg du sel disodique de l'acide éthylènediaminetétracétique (agent complexant). Après refroidissement entre +3°C et +4°C, on introduit sous agitation et sans dépasser la température de +4°C 7100, litres d'une solution d'hydrate d'hydrazine à 80 %. Cette introduction est faite en maintenant le pH (mesuré à +4°C) à 6 - 6,5 par introduction simultanée de 4549 litres d'acide formique 100 %.

b) La solution obtenue est transférée dans un réacteur équipé d'une agitation, d'une mesure de température, d'une mesure de pH installée sur une recirculation de la solution réactionnelle refroidie à +20°C, d'une introduction d'azote et d'un système d'échangeurs permettant de chauffer la solution à 80°C et de maitriser par refroidissement l'exothermicité se produisant vers 70°C pendant la montée en température.

On porte la température de la solution à 80°C et la maintient jusqu'à ce que la concentration en hydrate d'hydrazine soit inférieure à 1 g/litre. Le pH, mesuré à 20°C, est maintenu pendant ce temps à 7,6 par addition de 120 litres d'acide formique 100 %.

La solution de formiate d'aminoguanidine ainsi obtenue est ensuite transférée dans un bac tampon maintenu sous couverture d'azote. Elle représente un volume de 40900 l ou un poids de 44755 kg.

c) A partir de ce bac, on alimente un évaporateur en continu avec 1705 l/heure de solution de formiate d'aminoguanidine et 1585 l/heure des eaux-mères et des eaux de premier et deuxième lavages provenant d'une filtration antérieure de formiate d'aminoguanidine.

L'évaporation de ce mélange est conduite à environ 35°C sous un vide de 20 à 30 torr. La suspension obtenue en sortie de l'évaporateur a une concentration en solides voisine de 500 g/l et est transférée dans un cristalliseur maintenu à 20°C.

d) Le suspension (2185 kg/h) est ensuite filtrée, puis le solide est lavé trois fois avec:

. Premier lavage: 150 kg/h d'eaux provenant du troisième lavage,

. Deuxième lavage: 180 kg/h d'eau épurée,

. Troisième lavage: 75 kg/h d'eau épurée.

On obtient une production de 573 kg/h du formiate d'aminoguanidine à 5 % d'eau.

Les eaux-mères et les eaux issues des premier et deuxième lavage sont recyclées à l'évaporateur, tandis que les eaux issues du troisième lavage sont utilisées au premier lavage.

e) Dans un réacteur émaillé, muni d'une agitation, d'une mesure de température, d'une prise de vide et d'un échangeur capable d'assurer un chauffage à 160°C, on introduit 2749 kg de formiate d'aminoguanidine à 5 % d'eau obtenu précédemment. A la pression atmosphérique, on chauffe jusqu'à 120°C, température à laquelle se produit la fusion du formiate. En environ une heure et demie et sous un vide de 20 à 30 torr, on porte ensuite la température du mélange à 155 - 160°C. On ramène alors à la pression atmosphérique et maintient à 155 - 160°C pendant une heure et demie, puis on transfère le produit vers une écailleuse.

On obtient ainsi 1800 kg d'amino-3 triazole-1,2,4 présentant les caractéristiques suivantes:

. Titre: 97 - 98 %

. Teneur en eau: 0,15 %

. Filtrabilité: 5 - 10 secondes

. Floculat insoluble: 0

Pour évaluer la filtrabilité, on solubilise 30 g d'amino-3 triazole-1,2,4 dans 70 ml d'eau à 35°C, puis on filtre sous un vide 60 torr sur un filtre en fibre de verre (Whatman G F/C) de 70 mm de diamètre.

**Revendications**

1. Procédé de préparation de l'amino-3 triazole-1,2,4 qui consiste à faire réagir d'abord l'hydrate d'hydrazine, la cyanamide et l'acide formique, puis à cycliser séparément le formiate d'aminoguanidine, caractérisé en ce que:

a) on ajoute simultanément l'hydrate d'hydrazine et l'acide formique à la cyanamide de façon à maintenir la température entre 0 et 10°C et le pH (mesuré à 4°C) entre 6 et 7;

b) on porte ensuite le mélange à une température comprise entre 60 et 100°C et l'y maintient jusqu'à disparition de l'hydrate d'hydrazine, le pH (mesuré à 20°C) étant maintenu entre 7 et 8 par addition d'acide formique;

c) puis on évapore partiellement la solution de formiate d'aminoguanidine ainsi obtenue, à une température comprise entre 30 et 60°C jusqu'à ce que la teneur en solides soit comprise entre 100 et 700 g/l;

d) on filtre la suspension obtenue et lave le formiate d'aminoguanidine jusqu'à ce que sa teneur en dicyandiamide soit inférieure à 0,25 % en poids par rapport au formate sec, les eaux-mères et une partie ou la totalité des eaux de lavage étant recyclées à l'évaporation; et

e) on chauffe le formiate d'aminoguanidine ainsi obtenu à une température comprise entre 110 et 200°C.

2. Procédé selon la revendication 1, dans lequel l'évaporation est effectuée sous vide à une température comprise entre 35 et 45°C jusqu'à ce que la teneur en solides soit voisine de 500 g/l.

3. Procédé selon la revendication 1 ou 2, dans lequel on engage les réactifs dans un rapport molaire: hydrazine/cyanamide/acide formique allant de 1/1/1 à 1/1,05/1,08.

## Patentansprüche

1. Verfahren zur Herstellung von 3-Amino-1,2,4-triazol, das darin besteht, daß man zunächst Hydrazinhydrat, Cyanamid und Ameisensäure miteinander umsetzt und anschließend getrennt das Aminoguanidinformiat cyclisiert,

dadurch gekennzeichnet, daß man

a) gleichzeitig das Hydrazinhydrat und die Ameisensäure zu dem Cyanamid zugibt in der Art, daß die Temperatur zwischen 0 und 10°C und der pH-Wert (gemessen bei 4°C) zwischen 8 und 7 gehalten wird;

b) anschließend das Gemisch auf eine Temperatur zwischen 80 und 100°C bringt und dort hält bis zum Verschwinden des Hydrazinhydrats, wobei der pH-Wert (gemessen bei 20°C) durch Zugabe von Ameisensäure zwischen 7 und 8 gehalten wird;

c) anschließend die Lösung des so erhaltenen Aminoguanidinformiats bei einer Temperatur zwischen 30 und 80°C teilweise eindampft, bis der Feststoffgehalt zwischen 100 und 700 g/l liegt;

d) die erhaltene Suspension filtriert und das Aminoguanidin wäscht, bis der Gehalt an Dicyandiamid unter 0,25 Gew.-%, bezogen auf das trockene Formiat, liegt, wobei die Mutterlauge und ein Teil oder das gesamte Waschwascher zum Eindampfen zurückgeführt wird; und

e) das so erhaltene Aminoguanidinformiat auf eine Temperatur zwischen 110 und 200°C erhitzt.

2. Verfahren nach Anspruch 1, wobei das Eindampfen unter Vakuum bei einer Temperatur zwischen 35 und 45°C durchgeführt wird, bis der Feststoffgehalt nahe 500 g/l liegt.

3. Verfahren nach Anspruch 1 oder 2, wobei man die Reaktionspartner in einem Molverhältnis Hydrazin/Cyanamid/Ameisensäure von 1 : 1:1 bis 1 : 1,05 : 1,08 einsetzt.

## Claims

1. Process for the preparation of 3-amino-1,2,4-triazole, which consists in first reacting hydrazine hydrate, cyanamide and formic acid and in then separately cyclizing aminoguanidine formate, characterized in that:

a) hydrazine hydrate and formic acid are added simultaneously to cyanamide so as to maintain the temperature between 0 and 10°C and the pH (measured at 4°C) between 6 and 7;

b) the mixture is then heated to a temperature of between 60 and 100°C and is maintained thereat until the hydrazine hydrate has disappeared, the pH (measured at 20°C) being maintained between 7 and 8 by addition of formic acid;

c) the aminoguanidine formate solution thus obtained is then partially evaporated at a temperature of between 30 and 60°C until the solids content is between 100 and 700 g/l;

d) the suspension obtained is filtered and the aminoguanidine formate is washed until its dicyanodiamide content is below 0.25 % by weight relative to the dry formate, the mother liquors and a part or all of the wash waters being recycled to the evaporation; and

e) the aminoguanidine formate thus obtained is heated to a temperature of between 110 and 200°C.

2. Process according to Claim 1, in which the evaporation is carried out under vacuum at a temperature of between 35 and 45°C until the solids content is close to 500 g/l.

3. Process according to Claim 1 or 2, in which the reactants are used in a molar ratio: hydrazine/cyanamide/formic acid ranging from 1/1/1 to 1/1.05/1.08.